(19)

Europäisches
Patentamt
European
Patent Office
Office européen
des brevets

(11) **EP 2 899 181 A1**

(12) **EUROPEAN PATENT APPLICATION**

(43) Date of publication:
29.07.2015   Bulletin 2015/31

(51) Int Cl.:
*C07C 319/12* (2006.01)   *C07C 323/52* (2006.01)
*C08G 18/38* (2006.01)   *C08G 18/76* (2006.01)

(21) Application number: 15156037.2

(22) Date of filing: 12.04.2007

(84) Designated Contracting States:
DE FR GB IT

(30) Priority: 21.04.2006   JP 2006117641

(62) Document number(s) of the earlier application(s) in accordance with Art. 76 EPC:
07737057.5 / 2 011 784

(71) Applicant: Mitsui Chemicals, Inc.
Tokyo 105-7117 (JP)

(72) Inventors:
• Sakata, Michiharu
Omuta-shi, Fukuoka 836-8610 (JP)

• Kuma, Shigetoshi
Omuta-shi, Fukuoka 836-8610 (JP)
• Kobayashi, Seiichi
Omuta-shi, Fukuoka 836-8610 (JP)

(74) Representative: Wills, Andrew Jonathan et al
Mewburn Ellis LLP
City Tower
40 Basinghall Street
London EC2V 5DE (GB)

Remarks:
This application was filed on 20-02-2015 as a divisional application to the application mentioned under INID code 62.

(54) **Process for producing pentaerythritol mercaptocarboxylic esters and polymerizable compositions containing the esters**

(57)    A process for producing pentaerythritol mercaptocarboxylic ester, comprising purifying mercaptocarboxylic acid to reduce the content of thioester formed by condensation of two molecules of the acid to not more than 5% in terms of area percentage as determined at a wavelength of 230 nm by the high-performance liquid chromatography in the case of the total area of the mercaptocarboxylic acid and thioester formed by intermolecular condensation of the acid is taken as 100%, and reacting pentaerythritol with purified mercaptocarboxylic acid, wherein the content of bispentaerythritol in said pentaerythritol is not more than 7 wt %, based on the total weight of pentaerythritol.

EP 2 899 181 A1

**Description**

TECHNICAL FIELD

The present invention relates to a process for producing pentaerythritol mercaptocarboxylic esters, and polymerizable compositions composed of the pentaerythritol mercaptocarboxylic esters and polyiso(thio)cyanate compounds.

BACKGROUND ART

Plastic lenses are light weight, less broken, and dyeable, as compared with inorganic lenses. Therefore, in recent years, the application of the plastic lenses to optical materials for a spectacle lens, a camera lens, or the like has increased rapidly.

The resins for the plastic lenses have been required to have new excellent performances such as a high refractive index, a high Abbe's number, low specific gravity, and high heat resistance. A variety of resin materials for lenses have been developed and used until now.

Among them, there have been actively proposed polythiourethane-based resins, and the present inventors have been also proposed various plastic lenses obtained by using these polythiourethane-based resins (see Patent Documents 1, 2, and 3).

Among the polythiourethane-based resins, as the most typical resin, a polyurethane based resin obtained by polymerizing pentaerythritol mercaptocarboxylic ester with a polyiso(thio)cyanate compound is colorless and transparent, has a high refractive index and low dispersion, is excellent in impact resistance, dyeabiltiy, processability and the like, and is one of resins which are optimum for plastic lenses.

Pentaerythritol mercaptocarboxylic ester is produced by a so-called direct esterification method. For example, the ester is produced by reacting a usual polyhydric alcohol with a mercaptocarboxylic acid in the presence of an esterifying catalyst, while removing by-produced water out of the system (Refer to Patent Document 4).

Pentaerythritol, one of starting materials of the pentaerythritol mercaptocarboxylic ester, is usually produced by subjecting acetaldehyde and formaldehyde to condensation. The purity of the pentaerythritol obtained by the appropriate production process is about 90 wt %, and pentaerythritol contains various kinds of impurities. One of such impurities is bispentaerythritol that is a condensation of two molecules of formaldehyde of pentaerythritol. When this bispentaerythritol is contained in pentaerythritol in excess of a specific amount, it has been known that there might possibly be problems such that it is difficult to be released from a mold after completion of polymerization with a polyiso(thio)cyanate compound in some cases, and bubbles are generated inside the obtained lens (refer to Patent Documents 5 and 6).

With respect to such pentaerythritol, one of starting materials of pentaerythritol mercaptocarboxylic ester, there have been shown a correlation between its quality and impurities and the quality of the obtained lens in several documents. However, there has been scarcely known a correlation between the quality of the other starting material mercaptocarboxylic acid and the quality of the obtained lens.

As one of mercaptocarboxylic acids, a 3-mercaptopropionic acid can be cited. Since the 3-mercaptopropionic acid has extremely bad storage stability, it has been known that the purity is easily lowered due to the contact with oxygen in the air or storage temperature so that the content of impurities is increased. Furthermore, when the melting point of the 3-mercaptopropionic acid is low to be 16.8 degree centigrade, and the storage temperature becomes low particularly in winter or the like, the acid is solidified in some cases. Since a liquid is easily handled rather than a solid from the viewpoint of handling, the 3-mercaptopropionic acid is kept by heating so as not to be solidified or the acid is handled by heat-melting in advance when it is solidified in many cases. However, when the acid is excessively heated for storage by melting or heating, it causes a decrease in the purity. When pentaerythritol 3-mercaptopropionic ester is produced by using such a 3-mercaptopropionic acid and is employed for a long period of time, the quality of the obtained pentaerythritol 3-mercaptopropionic ester is not regular and the color is deteriorated in some cases even if the production conditions are the same. The viscosity of the polymerizable composition before polymerization obtained by mixing the pentaerythritol 3-mercaptopropionic ester with a polyiso (thio) cyanate compound is high so that it becomes difficult to handle the composition such that a) in the degassing step of the lens process, bubbles are hardly removed, b) in the filtering step for removing foreign substances, it takes time and filtering cannot be performed, c) injection into a mold cannot be done and the like. Furthermore, a lens obtained by the polymerizable composition has problems of deterioration in the color, whitening and the like.

Accordingly, it has been demanded that deterioration in the color of pentaerythritol mercaptocarboxylic ester, an increase in the viscosity of the polymerizable composition before polymerization with a polyiso(thio)cyanate compound, and deterioration in the color or whitening of the lens should be suppressed.

Patent Document 1: Japanese Patent Laid-open No. S60(1985)-199016
Patent Document 2: Japanese Patent Laid-open No. S60(1985)-217229

Patent Document 3: Japanese Patent Laid-open No. S63(1988)-46213
Patent Document 4: Japanese Patent Publication No. S39(1964)-9071
Patent Document 5: Japanese Patent Laid-open No. S56(1981)-20530
Patent Document 6: Japanese Patent Laid-open No. H10(1998)-120646

DISCLOSURE OF THE INVENTION

An object of the present invention is to obtain colorless and transparent pentaerythritol mercaptocarboxylic ester when pentaerythritol is reacted with a mercaptocarboxylic acid. Furthermore, the present invention is to provide a polymerizable composition having a low viscosity containing the pentaerythritol mercaptocarboxylic ester and polyiso(thio)cyanate, and to provide a polyurethane based resin which is colorless and transparent without causing whitening thereof by polymerizing the polymerizable composition.

In order to solve the above objects, the present inventors have conducted an extensive study and as a result, have determined that the cause of whitening of a polyurethane based resin is in the pentaerythritol mercaptocarboxylic ester as monomer thereof. The inventors have further continued an extensive study and as a result, surprisingly, when the pentaerythritol mercaptocarboxylic ester is produced using a mercaptocarboxylic acid having a content of thioester formed by condensation of two molecules of the mercaptocarboxylic acid of not more than a specific amount as a starting material, the above problems are solved, and a polyurethane based resin which is colorless and transparent and in which the whitening is suppressed is obtained. Thus, the present invention has been completed.

That is, the present invention relates to:

(1) a process for producing pentaerythritol mercaptocarboxylic ester, comprising;
reacting pentaerythritol with a mercaptocarboxylic acid that a content of thioester formed by condensation of two molecules of the acid is not more than 5% (in terms of area percentage) as determined by the high-performance liquid chromatography in the case of the total area of the mercaptocarboxylic acid and thioester formed by intermolecular condensation of the acid is taken as 100%;
(2) the process for producing pentaerythritol mercaptocarboxylic ester as set forth in (1) above, in which the content of bispentaerythritol in the pentaerythritol is not more than 7 wt %, based on the total weight of pentaerythritol;
(3) the process for producing pentaerythritol mercaptocarboxylic ester as set forth in (1) or (2) above, in which the mercaptocarboxylic acid is a 3-mercaptopropionic acid;
(4) a polymerizable composition containing the pentaerythritol mercaptocarboxylic ester obtained in accordance with the production process as set forth in any one of (1) to (3) above and a polyiso(thio)cyanate compound;
(5) a resin obtained by curing the polymerizable composition as set forth in (4) above;
(6) an optical material containing the resin as set forth in (5) above; and
(7) a lens containing the resin as set forth in (5) above.

Herein, in the aforementioned (4), a phrase "containing the pentaerythritol mercaptocarboxylic ester and a polyiso(thio)cyanate compound" refers to both a case in which the entire polymerizable composition is composed of the pentaerythritol mercaptocarboxylic ester and a polyiso(thio)cyanate compound and a case in which a part of the polymerizable composition is composed of the pentaerythritol mercaptocarboxylic ester and a polyiso(thio)cyanate compound. Meanwhile, in the above (6) and (7), a phrase "containing the resin" refers to both a case in which the entire optical material or the entire lens is composed of the resin and a case in which a part of the optical material or the lens is composed of the resin.

According to the production process of the present invention, colorless and transparent pentaerythritol mercaptocarboxylic ester is obtained. Furthermore, the polymerizable composition before polymerization obtained by mixing the pentaerythritol mercaptocarboxylic ester with a polyiso(thio)cyanate compound comes to have a low viscosity, while the polyurethane based resin obtained by polymerizing the polymerizable composition becomes a colorless and transparent resin in which whitening is suppressed.

BEST MODE FOR CARRYING OUT THE INVENTION

The present invention will be illustrated in detail below.
In the mercaptocarboxylic acid, as a starting material of pentaerythritol mercaptocarboxylic ester of the present invention, the content of thioester formed by condensation of two molecules of the mercaptocarboxylic acid is not more than a specific amount. Namely, there is used a mercaptocarboxylic acid that a content of thioester formed by condensation of two molecules of the acid is not more than 5% (in terms of area percentage) as determined by the high-performance liquid chromatography in the case of the total area of the mercaptocarboxylic acid and thioester formed by intermolecular condensation of the acid is taken as 100%.

Examples of the mercaptocarboxylic acid used in the present invention include a 3-mercaptopropionic acid, a 2-mercaptopropionic acid, a thioglycolic acid, a thiolactic acid, a thiomalic acid, a thiosalicylic acid and the like.

Herein, the thioester formed by intermolecular condensation of the mercaptocarboxylic acid is a compound obtained by condensation of a mercapto group and a carboxyl group of the mercaptocarboxylic acid between molecules by a thioester bond, and a compound bonded between two molecules, three or more molecules. The compound obtained by condensation of a mercapto group and a carboxyl group between two molecules by a thioester bond is referred to as thioester formed by condensation of two molecules. For example, thioester formed by intermolecular condensation of the 3-mercaptopropionic acid is a 3-(3-mercaptopropanoylthio)propionic acid.

When the content of thioester formed by condensation of two molecules of the mercaptocarboxylic acid is not more than 5% (in terms of area percentage) as determined by the high-performance liquid chromatography in the case of the total area of the mercaptocarboxylic acid and thioester formed by intermolecular condensation of the acid is taken as 100%, the color of the pentaerythritol mercaptocarboxylic ester produced by using the mercaptocarboxylic acid becomes colorless and transparent. Furthermore, the polymerizable composition before polymerization obtained by mixing the pentaerythritol mercaptocarboxylic ester with polyiso(thio)cyanate has a low viscosity, while the obtained polyurethane based resin becomes a colorless and transparent polyurethane based resin in which the whitening is suppressed. From the viewpoint of suppression of whitening, the content of thioester formed by condensation of two molecules of the mercaptocarboxylic acid used in the present invention is preferably from not less than 0.01% to not more than 5%, more preferably from not less than 0.01% to not more than 3%, and further preferably from not less than 0.01% to not more than 1% in terms of area percentage as determined by the high-performance liquid

chromatography.

The content of thioester formed by condensation of two molecules illustrated in the present invention is measured, for example, by the following method. A high-performance liquid chromatography system (LC-6A, SPD-10A, CTO-10A, products of Shimadzu Corporation) is connected with a column Mightysil RP-18 GP (a product of Kanto Chemical Co., Inc.) and an aqueous solution of 0.01M $KH_2PO_4$/acetonitrile (40/60) is used as an eluent, and the content of thioester formed by condensation of two molecules in the mercaptocarboxylic acid is analyzed at a column temperature of 40 degree centigrade at a flow rate of the eluent of 0.95 ml/min. at a wavelength of 230 nm. The content of thioester formed by condensation of two molecules is defined by area percentage as determined by the high-performance liquid chromatography in the case of the total area of the mercaptocarboxylic acid and thioester formed by intermolecular condensation of the acid is taken as 100%.

In the mercaptocarboxylic acid, the increased content of thioester formed by intermolecular condensation of the mercaptocarboxylic acid is caused by a method of storing the mercaptocarboxylic acid. The generation of thioester formed by intermolecular condensation of the mercaptocarboxylic acid is accelerated by the entrainment of iron in the mercaptocarboxylic acid, the contact of the mercaptocarboxylic acid and oxygen in the air, and when the storage temperature becomes high. Accordingly, the mercaptocarboxylic acid is preferably kept at a state that the temperature is controlled to be low in a vessel free from the contact with iron, in a nitrogen atmosphere. For example, the temperature suitable for the storage is in the range of not less than 10 to not more than 60 degree centigrade, more preferably in the range of not less than 15 to not more than 50 degree centigrade, and further preferably in the range of not less than 20 to not more than 40 degree centigrade.

Further, by means of purification, the content of thioester formed by condensation of two molecules in the mercaptocarboxylic acid may be reduced. The purification method is not particularly limited, but, for example, purification by distillation can be cited.

In the other starting material pentaerythritol, the content of bispentaerythritol of impurities and further the content of metals are preferably not more than a specific amount. For example, the content of bispentaerythritol in pentaerythritol is preferably in the range of not less than 0.01 to not more than 7 wt %, more preferably in the range of not less than 0.1 to not more than 5 wt %, and further preferably in the range of not less than 1 to not more than 5 wt %, based on the total weight of pentaerythritol.

Examples of the metal include alkali metals such as Li, Na, K, Rb, Cs and the like; alkaline earth metals such as Mg, Ca, Sr, Ba and the like; Ti, V, Cr, Mn, Fe, Co, Ni, Cu, Zn and the like. Specifically, it is preferable that the content of alkali metal and alkaline earth metal, particularly Na and Ca, is suppressed.

When the content of bispentaerythritol is within the aforementioned range and the total content of metals is less than 1 wt % based on the total weight of pentaerythritol, the release property from the mold after completion of the polymerization of the obtained pentaerythritol mercaptocarboxylic ester with polyiso(thio)cyanate becomes good so that the occurrence of bubbles in the obtained polyurethane based resin can be suppressed.

In order to react pentaerythritol with a mercaptocarboxylic acid, as the esterifying catalyst which is usually used, for example, acid catalysts having typical examples of mineral acids such as sulfuric acid, hydrochloric acid, phosphoric acid, alumina and the like; and organic acids such as p-toluenesulfonic acid, benzenesulfonic acid, methanesulfonic acid, trichloroacetic acid, dibutyl tin dioxide and the like are preferably used.

The preferable proportion of pentaerythritol and the mercaptocarboxylic acid is not particularly limited, but the molar ratio is, for example, in the range of not less than 3.5 to not more than 6.0 (mercaptocarboxylic acid/pentaerythritol), more preferably in the range of not less than 3.8 to not more than 5.0, and further preferably in the range of not less than 4.0 to not more than 4.5. When the proportion is within the above range, it is possible to produce pentaerythritol mercaptocarboxylic ester having high purity with good efficiency. The obtained pentaerythritol mercaptocarboxylic ester is colorless and transparent, and comes to have a low viscosity, and the polymerizable composition containing the pentaerythritol mercaptocarboxylic ester and a polyiso(thio)cyanate compound also comes to have a low viscosity. The resin obtained by curing the polymerizable composition is excellent in the color, and has excellent qualities in optical properties, heat resistance or the like.

Meanwhile, as the preferable condition for the reaction of pentaerythritol with a mercaptocarboxylic acid, for example, the temperature is in the range of not less than 80 to not more than 140 degree centigrade, and more preferably in the range of not less than 100 to not more than 125 degree centigrade. When the temperature is within the above range, the reaction of pentaerythritol with a mercaptocarboxylic acid is further accelerated. The obtained pentaerythritol mercaptocarboxylic ester is colorless and transparent, and comes to have a low viscosity, while the polymerizable composition containing the pentaerythritol mercaptocarboxylic ester and a polyiso(thio)cyanate compound also comes to have a low viscosity. The resin obtained by curing the polymerizable composition is excellent in the color, and has excellent qualities in optical properties, heat resistance or the like.

To produce pentaerythritol mercaptocarboxylic ester, an azeotropic agent is not necessarily used. However, there is generally used a method including continuously removing by-produced water out of the system under heating reflux using an azeotropic agent. Examples of the azeotropic agent which is usually used include benzene, toluene, xylene, nitrobenzene, chlorobenzene, dichlorobenzene, anisole, diphenyl ether, methylene chloride, chloroform, dichloroethane and the like. These may be used singly, or two or more kinds thereof may be used in combination, or may be used in mixture with other solvents.

The pentaerythritol mercaptocarboxylic ester of the present invention obtained by the aforementioned process is not particularly limited as long as it is a compound obtained by condensation of pentaerythritol with a mercaptocarboxylic acid. Examples thereof include the following compounds: pentaerythritol thioglycolic ester, pentaerythritol 3-mercaptopropionic ester, pentaerythritol thiolactic ester, pentaerythritol thiosalicylic ester and the like. Furthermore, these ester compounds may be compounds obtained by fully esterifying a hydroxy group of pentaerythritol or compounds obtained by esterifying only a part of a hydroxy group. Further, these ester compounds may be used singly, or two or more kinds thereof may be used in combination when a polyurethane based resin is obtained by polymerizing the ester compound with a polyiso(thio)cyanate compound.

The polyiso(thio)cyanate compound of the present invention is not particularly limited as long as it is a compound having at least two or more iso(thio)cyanate groups in a molecule. Examples thereof include aliphatic polyisocyanate compounds such as hexamethylene diisocyanate, 2,2-dimethylpentane diisocyanate, 2,2,4-trimethylhexane diisocyanate, butene diisocyanate, 1,3-butadiene-1,4-diisocyanate, 2,4,4-trimethylhexamethylene diisocyanate, 1,6,11-undecane triisocyanate, 1,3,6-hexamethylene triisocyanate, 1,8-diisocyanato-4-isocyanatomethyloctane, bis(isocyanatoethyl)carbonate, bis(isocyanatoethyl)ether, lysine diisocyanatomethyl ester, lysine triisocyanate and the like; polyisocyanate compounds having an aromatic compound such as 1,2-diisocyanatobenzene, 1,3-diisocyanatobenzene, 1,4-diisocyanatobenzene, 2,4-diisocyanatotoluene, ethylphenylene diisocyanate, isopropylphenylene diisocyanate, dimethylphenylene diisocyanate, diethylphenylene diisocyanate, diisopropylphenylene diisocyanate, trimethylbenzene triisocyanate, benzene triisocyanate, biphenyl diisocyanate, toluidine diisocyanate, 4,4'-methylenebis(phenyl isocyanate), 4,4'-methylenebis(2-methylphenyl isocyanate), bibenzyl-4,4'-diisocyanate, bis(isocyanatophenyl)ethylene, bis(isocyanatoethyl)benzene, bis(isocyanatopropyl)benzene, α,α,α',α'-tetramethylxylylene diisocyanate, bis(isocyanatobutyl)benzene, bis(isocyanatomethyl)naphthalene, bis(isocyanatomethylphenyl)ether, bis(isocyanatoethyl)phthalate, 2,6-di(isocyanatomethyl)furan and the like; sulfur-containing aliphatic polyisocyanate compounds such as bis(isocyanatomethyl)sulfide, bis(isocyanatoethyl)sulfide, bis(isocyanatopropyl)sulfide, bis(isocyanatohexyl)sulfide, bis(isocyanatomethyl)sulfone, bis(isocyanatomethyl)disulfide, bis(isocyanatoethyl)disulfide, bis(isocyanatopropyl)disulfide, bis(isocyanatomethylthio)methane, bis(isocyanatoethylthio)methane, bis(isocyanatomethylthio)ethane, bis(isocyanatoethylthio)ethane, 1,5-diisocyanato-2-isocyanatomethyl-3-thiapentane, 1,2,3-tris(isocyanatomethylthio)propane, 1,2,3-tris(isocyanatoethylthio)propane, 3,5-dithia-1,2,6,7-heptane tetraisocyanate, 2,6-diisocyanatomethyl-3,5-dithia-1,7-heptane diisocyanate, 2,5-diisocyanate methyl thiophene, 4-isocyanatoethylthio-2,6-dithia-1,8-octane diisocyanate and the like; aromatic sulfide based polyisocyanate compounds such as 2-isocyanatophenyl-4-isocyanatophenyl sulfide, bis(4-isocyanatophenyl)sulfide, bis(4-isocyanatomethylphenyl)sulfide and the like; aromatic disulfide based polyisocyanate compounds such as bis(4-isocyanatophenyl)disulfide, bis(2-methyl-5-isocyanatophenyl)disulfide, bis(3-methyl-5-isocyanatophenyl)disulfide, bis(3-methyl-6-isocyanatophenyl)disulfide, bis(4-methyl-5-isocyanatophenyl)disulfide, bis(4-methoxy-3-isocyanatophenyl)disulfide and the like; sulfur-containing alicyclic polyisocyanate compounds such as 2,5-diisocyanatotetrahydrothiophene, 2,5-diisocyanatomethyltetrahydrothiophene, 3,4-diisocyanatomethyltetrahydrothiophene, 2,5-diisocyanato-1,4-dithiane, 2,5-diisocyanatomethyl-1,4-dithiane, 4,5-diisocyanato-1,3-dithiolane, 4,5-bis(isopyanatomethyl)-1,3-dithiolane, 4,5-diisocyanatomethyl-2-methyl-1,3-dithiolane and the like; aliphatic polyisothiocyanate compounds such as 1,2-diisothiocyanatoethane, 1,6-diisothiocyanatohexane and the like; alicyclic polyisothiocyanate compounds such as cyclohexane diisothiocyanate and the like; aromatic polyisothiocyanate compounds such as 1,2-diisothiocyanatobenzene, 1,3-diisothiocyanatobenzene, 1,4-diisothiocyanatobenzene, 2,4-diisothiocyanatotoluene, 2,5-diisothiocyanato-m-xylene, 4,4'-methylenebis(phenyl isothiocyanate), 4,4'-methylenebis(2-methylphenyl isothiocyanate), 4,4'-methylenebis(3-methylphenyl isothiocyanate), 4,4'-diisothiocyanatobenzophenone, 4,4'-diisothiocyanato-3,3'-dimethylbenzophenone, bis(4-isothiocyanatophenyl)ether and the like; further, carbonyl polyisothiocyanate compounds such as 1,3-benzenedicarbonyl diisothiocyanate, 1,4-benzenedicarbonyl diisothiocyanate, (2,2-pyridine)-4,4-dicarbonyl diisothiocyanate and the like; sulfur-containing aliphatic polyisothiocyanate compounds such as thiobis(3-isothiocyanatopropane), thiobis(2-isothiocyanatoethane), dithiobis(2-isothiocyanatoethane) and the like; sulfur-containing aromatic polyisothiocyanate compounds such as 1-isothiocyanato-4-[(2-isothiocyanato)sulfonyl]benzene, thiobis(4-isothiocyanatobenzene), sulfonyl(4-isothiocyanatobenzene), dithiobis(4-isothiocyanatobenzene) and the like; sulfur-containing alicyclic polyisothiocyanate compounds such as 2,5-diisothiocyanatothiophene, 2,5-diisothiocyanato-1,4-dithiane and the like; and compounds having an isocyanato group and an isothiocyanato group such as 1-isocyanato-6-isothiocyanatohexane, 1-isocyanato-4-isothiocyanatocyclohexane, 1-isocyanato-4-isothiocyanatobenzene, 4-methyl-3-isocyanato-1-isothiocyanatobenzene, 2-isocyanato-4,6-diisothiocyanato-1,3,5-triazine, 4-isocyanatophenyl-4-isothiocyanatophenyl sulfide, 2-isocyanatoethyl-2-isothiocyanatoethyl disulfide and the like.

Furthermore, there can be used their halogen substituted compounds such as chlorine substituted compounds, bromine substituted compounds or the like, their alkyl substituted compounds, their alkoxy substituted compounds, their nitro substituted compounds, prepolymer type modified compounds modified with polyhydric alcohols, carbodiimide-modified compounds, urea-modified compounds, biuret-modified compounds, compounds obtained by dimerization or trimerization reaction or the like. These compounds may be used singly, or two or more compounds may be used in combination.

The proportion of the pentaerythritol mercaptocarboxylic ester and the polyiso (thio) cyanate compound is not particularly limited, but the molar ratio is usually in the range of not less than 0.3 to not more than 2.0 (SH group/NCO group), preferably in the range of not less than 0.7 to not more than 2.0, and further preferably in the range of not less than 0.8 to not more than 1.3. When the molar ratio is within the above range, the resin obtained by curing the polymerizable composition containing the pentaerythritol mercaptocarboxylic ester and the polyiso(thio)cyanate compound is excellent in the color, and has excellent qualities in optical properties, heat resistance or the like.

For purposes of improvement of general properties, operability, polymerization reactivity and the like of the polyurethane based resin of the present invention, other substances may be added, in addition to the ester compound and iso(thio)cyanate compound forming the urethane resin. For example, in addition to a starting material for forming an urethane, one or two or more active hydrogen compounds having typical examples of amine and the like, epoxy compounds, olefin compounds, carbonate compounds, ester compounds, metals, metal oxides, organic metal compounds, inorganic substances or the like may be added.

Further, a variety of substances such as a chain extender, a crosslinking agent, a photostabilizer, a UV absorber, an antioxidant, an oil soluble dye, a filler, a releasing agent, and a blueing agent, may be added, depending on the purposes, as in a known molding method. In order to adjust to a desired reaction rate, a thiocarbamic acid S-alkyl ester or a known reaction catalyst used for producing polyurethane may be added as appropriate. The lens formed of the polyurethane resin of the present invention can be usually obtained by casting polymerization.

Specifically, pentaerythritol mercaptocarboxylic ester is mixed with a polyiso(thio)cyanate compound. This mixed solution is degassed according to a proper method as needed, and then injected into a mold and usually slowly heated from a low temperature to a high temperature for polymerization.

The thus-obtained polyurethane based resin of the present invention has a high refractive index, a low dispersion, excellent heat resistance and durability, light weight, and excellent impact resistance and the occurrence of whitening is further suppressed. Thereby it being suitable as an optical material and a transparent material for a spectacle lens, a camera lens, or the like.

Furthermore, the lens which is obtained by using the polyurethane resin of the present invention may be, if necessary, subjected to physical or chemical treatment such as surface abrasion treatment, antistatic treatment, hard coat treatment, non-reflective coat treatment, dyeing treatment and polarizing treatment, for prevention of reflection, enhancement of hardness, improvement of abrasion resistance, improvement of chemical resistance, supply of anticlouding, supply of fashionability, and the like.

EXAMPLES

The present invention is now illustrated in detail below with reference to Examples. In the following Examples and Comparative Examples, as a mercaptocarboxylic acid, a 3-mercaptopropionic acid was used. The 3-mercaptopropionic acid was analyzed by the following method. The color of the obtained pentaerythritol 3-mercaptopropionic ester, the viscosity of a polymerizable composition before polymerization composed of pentaerythritol 3-mercaptopropionic ester and a polyiso(thio)cyanate compound, and the color and transparency of a polyurethane based resin obtained by polymerization were evaluated in the following test method.

· Content of 3-(3-mercaptopropanoylthio)propionic acid: A high-performance liquid chromatography system (LC-6A, SPD-10A, CTO-10A, products of Shimadzu Corporation) was connected with a column Mightysil RP-18GP (a product of Kanto Chemical Co., Inc.) and the content thereof was measured by the high-performance liquid chromatography. Specifically, using an aqueous solution of 0.01M $KH_2PO_4$/acetonitrile (40/60) as an eluent and dissolving the 3-mercaptopropionic acid in the eluent, area percentage of the 3-(3-mercaptopropanoylthio)propionic acid was analyzed at a column temperature of 40 degree centigrade at a flow rate of the eluent of 0.95 ml/min. at a wavelength of 230 nm in the case of the total area of the mercaptocarboxylic acid and thioester formed by intermolecular condensation of the acid was taken as 100%.

· Content of bispentaerythritol: Pentaerythritol was dissolved in water, and then the resulting aqueous solution was applied to the high-performance liquid chromatography to measure the content of bispentaerythritol.

· Content of sodium and calcium: Pentaerythritol was dissolved in water, and then the resulting aqueous solution was applied to the high-performance liquid ion chromatography to measure the content of sodium and calcium.

· Y.I. (yellow index) of pentaerythritol mercaptocarboxylic ester: Y.I. was employed as an analyzing item for evaluating the color of the pentaerythritol mercaptocarboxylic ester. The smaller the Y.I. value was, the better the color of the pentaerythritol mercaptocarboxylic ester was, while the greater the Y.I. value was, the worse the color was. Such a correlation was obtained. Using a colorimeter CT-210 (a product of Minolta Camera Co., Ltd.), tristimulus value Y and color coordination x, y on the CIE-1391 chromaticity diagram were measured. Firstly, distilled water was fed into a cell CT-A20 having an optical path length of 20 mm, and a white calibration was performed as Y=100.00, x=0.3101 and y=0.3162. Thereafter, a sample was fed into the same cell and the color measurement was carried out. The measurement results, x and y values, were used to calculate Y.I. according to the following formula:

$$Y.I. = (234 \times x + 106 \times y + 106)/y \qquad (1)$$

· Y.I. (yellow index) of polyurethane based resin: Y.I. was employed as an analyzing item for evaluating the color of a plastic lens containing a polyurethane based resin. The smaller the Y.I. value was, the better the color of the plastic lens was, while the greater the Y.I. value was, the worse the color was. Such a correlation was obtained. The plastic lens of a circular flat plate having a thickness of 9 mm and $\varphi$75 mm was prepared, and chromaticity coordinates x and y were measured by using a colorimeter CT-210 (a product of Minolta Camera Co., Ltd.). The measurement results, x and y values, were used to calculate Y.I. according to the above formula (1).

· Loss degree of transparency: As an analyzing item for evaluating the transparency of the plastic lens containing a polyurethane based resin, the loss degree of transparency was employed. The loss degree of transparency was obtained in the following means. The lens plate of a circular flat plate having a thickness of 9 mm and $\varphi$75 mm was prepared. Then, the lens plate was irradiated with a light source (Luminar Ace LA-150A, a product of Hayashi Watch Works Co., Ltd.) for measuring the loss degree of transparency with a gray scale image processing unit. Captured images were expressed in numbers by gray scale image processing to obtain the loss degree of transparency. When the loss degree of transparency is not more than 50, it was indicated with ○, while, when it was greater than 50, it was indicated with ×.

Example 1

Synthesis of pentaerythritol 3-mercaptopropionic ester

To a 2-liter, 4-necked flask equipped with a stirrer, a reflux condensing water separator, a nitrogen gas purge tube and a thermometer were added 663.0 parts by weight (6.23 mol) of a 3-mercaptopropionic acid with the purity of 99.7% containing 0.2% (in terms of area percentage) of 3-(3-mercaptopropanoylthio)propionic acid, 204.6 parts by weight (1.5 mol) of pentaerythritol with the purity of 95.2% containing 4.7 wt % of bispentaerythritol, 0.1 wt % of sodium and 0.02 wt % of calcium, 5.7 parts by weight of p-toluenesulfonic acid·monohydrate and 292.5 parts by weight of toluene. While by-produced water was continuously removed out of the system under heating reflux, the resulting solution was reacted for 7.0 hours (internal temperature of 96 to 121 degree centigrade), and then cooled down to room temperature. The amount of water removed out of the system was 99.3% based on the theoretical amount of water to be generated. The reaction solution was washed with a base and subsequently washed with water, and then the reaction solution is removed toluene and a trace of water under heat and reduced pressure. Thereafter, 716.8 parts by weight of pentaerythritol 3-mercaptopropionic ester was obtained by filtering. Y.I. of the obtained pentaerythritol 3-mercaptopropionic ester was 1.0.

Viscosity of polymerizable composition before polymerization 87 parts by weight of m-xylylene diisocyanate, 0.01 weight part of dibutyltin dichloride as a curing catalyst, 0.18 parts by weight of ZELEC UN (product name, acid phosphate ester, a product of Stepan Co.) and 0. 10 weight part of Viosorb 583 (product name, ultraviolet absorber, a product of Kyodo Chemical Co., Ltd.) were mixed and dissolved at 20 degree centigrade. 113 parts by weight of the obtained pentaerythritol 3-mercaptopropionic ester was fed thereinto and mixed to give a uniform polymerizable composition before polymerization. The polymerizable composition before polymerization was kept at 20 degree centigrade and stirred for 7.0 hours. The viscosity at that time was 157 mPa·s.

Production of plastic lens

87 parts by weight of m-xylylene diisocyanate, 0.01 weight part of dibutyltin dichloride as a curing catalyst, 0.18 parts by weight of ZELEC UN (product name, acid phosphate ester, a product of Stepan Co.) and 0.10 weight part of Viosorb 583 (product name, ultraviolet absorber, a product of Kyodo Chemical Co., Ltd.) were mixed and dissolved at 20 degree centigrade. 113 parts by weight of the obtained pentaerythritol 3-mercaptopropionic ester was fed thereinto and mixed to give a uniform polymerizable composition before polymerization. The polymerizable composition before polymerization was degassed at 600 Pa for 1 hour, and then filtered using a 3-μm PTFE filter. Thereafter, the resulting solution was injected into a mold equipped with a glass mold and tapes. This mold was put into an oven and then gradually heated from 10 to 120 degree centigrade at which polymerization was conducted for 18 hours. After completion of polymerization, the mold was taken out from the oven and a resin was released from the mold. The obtained resin was additionally annealed at 130 degree centigrade for 4 hours. Y.I. of the obtained resin was 3.7 and the loss degree of transparency exhibiting transparency was 22. So, the resin was indicated with o regarding the loss degree of transparency.

Example 2

pentaerythritol 3-mercaptopropionic ester was synthesized in the same manner as in Example 1, except that a 3-mercaptopropionic acid with the purity of 96.1% containing 3.4% (in terms of area percentage) of 3-(3-mercaptopropanoylthio)propionic acid was used instead of the 3-mercaptopropionic acid used in Example 1. Y.I. of the obtained pentaerythritol 3-mercaptopropionic ester was 1.3. The viscosity of a polymerizable composition before polymerization with m-xylylene diisocyanate, which is containing the obtained pentaerythritol 3-mercaptopropionic ester and is obtained in the same manner as in Example 1, was 248 mPa·s. Furthermore, a plastic lens was prepared in the same manner as in Example 1. The evaluation results of the obtained plastic lens are shown in Table 1.

Example 3

pentaerythritol 3-mercaptopropionic ester was synthesized in the same manner as in Example 1, except that a 3-mercaptopropionic acid with the purity of 95.3% containing 4.2% (in terms of area percentage) of 3-(3-mercaptopropanoylthio)propionic acid was used instead of the 3-mercaptopropionic acid used in Example 1. Y.I. of the obtained pentaerythritol 3-mercaptopropionic ester was 1.8. The obtained pentaerythritol 3-mercaptopropionic ester was used and the viscosity of a polymerizable composition before polymerization with m-xylylene diisocyanate obtained in the same manner as in Example 1 was 288 mPa·s. Furthermore, a plastic lens was prepared in the same manner as in Example 1. The evaluation results of the obtained plastic lens are shown in Table 1.

Comparative Example 1

pentaerythritol 3-mercaptopropionic ester was synthesized in the same manner as in Example 1, except that a 3-mercaptopropionic acid with the purity of 90.2% containing 7.5% (in terms of area percentage) of 3-(3-mercaptopropanoylthio)propionic acid was used instead of the 3-mercaptopropionic acid used in Example 1. Y.I. of the obtained pentaerythritol 3-mercaptopropionic ester was 3.3. The obtained pentaerythritol 3-mercaptopropionic ester was used and the viscosity of a polymerizable composition before polymerization with m-xylylene diisocyanate obtained in the same manner as in Example 1 was 380 mPa·s. Furthermore, a plastic lens was prepared in the same manner as in Example 1. The evaluation results of the obtained plastic lens are shown in Table 1.

Comparative Example 2

pentaerythritol 3-mercaptopropionic ester was synthesized in the same manner as in Example 1, except that a 3-mercaptopropionic acid with the purity of 87.5% containing 11.1% (in terms of area percentage) of 3-(3-mercaptopropanoylthio)propionic acid was used instead of the 3-mercaptopropionic acid used in Example 1. Y.I. of the obtained pentaerythritol 3-mercaptopropionic ester was 5.2. The obtained pentaerythritol 3-mercaptopropionic ester was used and the viscosity of a polymerizable composition with m-xylylene diisocyanate obtained in the same manner as in Example 1 was 2,000 mPa·s or more. Furthermore, a plastic lens was attempted to be prepared in the same manner as in Example 1 but as a result, a plastic resin could not be obtained. This was because, since the viscosity of a polymerizable composition before polymerization was unusually high, filtering by using a 3-$\mu$m PTFE filter was extremely slow and it was difficult to inject the polymerizable composition into a mold equipped with a glass mold and tapes.

[Table 1]

| | Content of thioester (%) | Content of bispentaerythritol (wt %) | Viscosity of polymerizable composition (mPa·s) | Evaluation of thiol | Evaluation of lens | |
| --- | --- | --- | --- | --- | --- | --- |
| | | | | Color Y.I. | Color Y.I. | Transparency Loss degree of transparency ($\leq$ 50) |
| Example 1 | 0.2 | 4.7 | 157 | 1.0 | 3.7 | ○ (22) |
| Example 2 | 3.4 | 4.7 | 248 | 1.3 | 4.0 | ○ (39) |
| Example 3 | 4.2 | 4.7 | 288 | 1.8 | 4.0 | ○ (26) |
| Comparative Example 1 | 7.5 | 4.7 | 380 | 3.3 | 5.8 | ✕ (65) |
| Comparative Example 2 | 11.1 | 4.7 | 2,000 or more | 5.2 | Unable to measure | Unable to measure |

INDUSTRIAL APPLICABILITY

According to the present invention, a polymerizable composition which is easily subjected to degassing, filtering of foreign substances and injection into a mold is obtained. Furthermore, using such a polymerizable composition, a high-quality urethane based plastic resin having excellent optical properties can be more economically produced.
Embodiments of the invention are described in the numbered paragraphs below.

1. A process for producing pentaerythritol mercaptocarboxylic ester, comprising:

reacting pentaerythritol with a mercaptocarboxylic acid that a content of thioester formed by condensation of two molecules of the acid is not more than 5% in terms of area percentage as determined by the high-performance liquid chromatography in the case of the total area of the mercaptocarboxylic acid and thioester formed by intermolecular condensation of the acid is taken as 100%.

2. The process for producing pentaerythritol mercaptocarboxylic ester as set forth in 1, in which the content of bispentaerythritol in said pentaerythritol is not more than 7 wt %, based on the total weight of pentaerythritol.

3. The process for producing pentaerythritol mercaptocarboxylic ester as set forth in 1 or 2, in which said mercaptocarboxylic acid is a 3-mercaptopropionic acid.

4. A polymerizable composition comprising the pentaerythritol mercaptocarboxylic esters obtained in accordance with the production process as set forth in any one of 1 to 3 and a polyiso(thio)cyanate compound.

5. A resin obtained by curing the polymerizable composition as set forth in 4.

6. An optical material comprising the resin as set forth in 5.

7. A lens comprising the resin as set forth in 5.

**Claims**

1. A process for producing pentaerythritol mercaptocarboxylic ester, comprising:

   purifying mercaptocarboxylic acid to reduce the content of thioester formed by condensation of two molecules of the acid to not more than 5% in terms of area percentage as determined at a wavelength of 230 nm by the high-performance liquid chromatography in the case of the total area of the mercaptocarboxylic acid and thioester formed by intermolecular condensation of the acid is taken as 100% and
   reacting pentaerythritol with purified mercaptocarboxylic acid,
   wherein the content of bispentaerythritol in said pentaerythritol is not more than 7 wt %, based on the total weight of pentaerythritol.

2. The process for producing pentaerythritol mercaptocarboxylic ester as set forth in claim 1, wherein said mercaptocarboxylic acid is selected from group consisting of a 2-mercaptopropionic acid, a thioglycolic acid, a thiolactic acid, a thiomalic acid and a thiosalicylic acid.

3. A polymerizable composition comprising the pentaerythritol mercaptocarboxylic esters obtained in accordance with the production process as set forth in claim 1 or 2 and a polyiso(thio)cyanate compound.

4. A resin obtained by curing the polymerizable composition as set forth in claim 3.

5. An optical material comprising the resin as set forth in claim 4.

6. A lens comprising the resin as set forth in claim 4.

**EP 2 899 181 A1**

Europäisches
Patentamt
European
Patent Office
Office européen
des brevets

**EUROPEAN SEARCH REPORT**

Application Number

EP 15 15 6037

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2001 039945 A (MITSUI CHEMICALS INC) 13 February 2001 (2001-02-13) | 3-6 | INV. C07C319/12 C07C323/52 C08G18/38 C08G18/76 |
| Y | * paragraphs [0027] - [0028]; claims 1, 6 * | 1,2 | |
| X | JP 2005 336104 A (MITSUI CHEMICALS INC) 8 December 2005 (2005-12-08) | 3-6 | |
| Y | * paragraphs [0014] - [0015], [0018]; claims 1, 5-8 * | 1,2 | |

TECHNICAL FIELDS
SEARCHED       (IPC)

C07C
C08G

The present search report has been drawn up for all claims

| Place of search | Date of completion of the search | Examiner |
|---|---|---|
| The Hague | 1 June 2015 | Mooren, Nicolai |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

...........................................................................................
& : member of the same patent family, corresponding
document

EPO FORM 1503 03.82 (P04C01)

11

## ANNEX TO THE EUROPEAN SEARCH REPORT
## ON EUROPEAN PATENT APPLICATION NO.

EP 15 15 6037

This annex lists the patent family members relating to the patent documents cited in the above-mentioned European search report.
The members are as contained in the European Patent Office EDP file on
The European Patent Office is in no way liable for these particulars which are merely given for the purpose of information.

01-06-2015

| Patent document cited in search report | | Publication date | Patent family member(s) | | Publication date |
|---|---|---|---|---|---|
| JP 2001039945 | A | 13-02-2001 | JP<br>JP | 4426018 B2<br>2001039945 A | 03-03-2010<br>13-02-2001 |
| JP 2005336104 | A | 08-12-2005 | JP<br>JP | 4339181 B2<br>2005336104 A | 07-10-2009<br>08-12-2005 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S601985199016 B
- JP S601985217229 B
- JP S63198846213 B
- JP S3919649071 B
- JP S56198120530 B
- JP H101998120646 B